(19) Europäisches Patentamt
European Patent Office
Office européen des brevets

(11) Publication number: **0 026 090**
**B1**

(12) # EUROPEAN PATENT SPECIFICATION

(45) Date of publication of patent specification: **14.09.83**

(21) Application number: **80303293.7**

(22) Date of filing: **18.09.80**

(51) Int. Cl.$^3$: **C 01 B 25/32, C 04 B 35/00, A 61 F 1/00**

(54) **Process for preparing hydroxyapatite, and filter cake, ceramic material and implant material comprising hydroxyapatite.**

(30) Priority: **25.09.79 JP 123000/79**

(43) Date of publication of application: **01.04.81 Bulletin 81/13**

(45) Publication of the grant of the patent: **14.09.83 Bulletin 83/37**

(84) Designated Contracting States: **DE FR GB IT NL**

(56) References cited:
**DE - A - 1 667 836**
**US - A - 3 379 541**
**US - A - 4 097 935**

**ANNALES DE CHIMIE, tome 7, 1952 Paris, FR R. WALLAEYS: "Contribution à l'étude des apatites phosphocalciques," pages 808—848**

(73) Proprietor: **KUREHA KAGAKU KOGYO KABUSHIKI KAISHA**
**9-11 Horidome-cho 1-chome Nihonbashi Chuo-ku Tokyo (JP)**

(72) Inventor: **Nagai, Hirosi**
**4-2-47, Fujimi-cho Chofu-shi Tokyo (JP)**
Inventor: **Nishimura, Yasushi No. 404 Haitsu-Takada-No-Baba**
**116-11 Takada-No-Baba 1-chome Shinjuku-ku Tokyo (JP)**

(74) Representative: **Myerscough, Philip Boyd et al, J.A.Kemp & Co. 14, South Square Gray's Inn London, WC1R 5EU (GB)**

**EP 0 026 090 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Process for preparing hydroxyapatite, and filter cake, ceramic material and implant material comprising hydroxyapatite

The present invention relates to a process for preparing hydroxyapatite, and to a filter cake, ceramic material or implant material comprising hydroxyapatite. The present invention is primarily concerned with providing a colourless and translucent ceramic material of hydroxyapatite having a high density, a high purity and a high thermal stability, which is useful in implant materials comprising a ceramic material and an organic binding material.

Hydroxyapatite is represented by the formula $Ca_{10}(PO_4)_6(OH)_2$ or $Ca_5(PO_4)_3(OH)$, and is an inorganic component of the hard tissues of living bodies such as bones, and teeth.

It is known that sintered synthetic hydroxyapatite (hereinafter referred to as a ceramic hydroxyapatite material) is usable as an implant material, such as an artificial dental root or artificial bone (for instance, see Aoki and Kato, "Ceramic", 10(7): 469 (1975)). Clearly a ceramic hydroxyapatite material should resemble naturally occurring hydroxyapatite in its physical properties. THus, a ceramic hydroxyapatite material for use in a living body should be colourless, have a high density and purity and be safe to use.

A pure ceramic material should not contain, as far as possible, elements other than Ca, P, O (as $PO_4$) and H (as OH), the elemental components of hydroxyapatite: its Ca/P atomic ratio should be close to 1.67/1 which is the theoretical value of hydroxyapatite and the physical structure should be as close as possible to that of pure hydroxyapatite. Defects in the physical structure of a ceramic material, if any, can be detected by X-ray analysis or by treating a specimen at a high temperature of about 1350°C. The latter method, as will subsequently be explained in more detail, readily detects the presence of an impurity which is frequently contained in a synthetic ceramic hydroxyapatite material, namely whitlockite which has a lower Ca/P value and a different physical structure than natural hydroxyapatite and is highly soluble in aqueous solvents.

The presence of impurities and structural defects in a synthetic ceramic material makes the material coloured and the impurities tend to be eluted within a living body containing an implant of the synthetic material. According to one viewpoint, it may be preferable to have an accelerated elution of impurities within the tissues of the living body, but from the viewpoint of safety there may be unfavorable effects on the living body due to topical electrolyte imbalance or harmful action of an impurity upon such elution.

Accordingly, there is a need for a synthetic ceramic hydroxyapatite material that is highly pure, almost colourless and of high density. However, as will be explained below, a synthetic ceramic material satisfying such demands had not previously been known.

A number of processes for synthesizing hydroxyapatite have been proposed, for instance, by R. W. Mooney et al., "Chem. Rev.", 61:433(1961) and by Kanazawa, et al., "Kagaku no Ryoiki", 27:622(1973). However, there are few disclosures of the preparation of a ceramic material by the firing of a synthetic hydroxyapatite. Monreo has reported that he obtained a ceramic material by compression-molding powdery hydroxyapatite and firing the molded material at a temperature of 1300°C under normal pressure (refer to "J. Dent. Res.", 50,860 (1971)), but since his ceramic material contains as much as 30% by weight of whitlockite (alpha-calcium triphosphate), it cannot be said that his ceramic material is of highly pure hydroxyapatite.

A reason why a ceramic material of highly pure hydroxyapatite has not been available is considered to be the fact that the hydroxyapatite used for making the ceramic material did not have the correct stoichiometrical composition or it had some structural defects resulting in the by-production of whitlockite at the time of firing. To test this, a trial has been carried out wherein hydroxyapatite of the correct stoichiometric composition was synthesized and then the hydroxyapatite was fired. However, the hydroxyapatite synthesized by the conventional process was difficult to sinter and of poor thermal stability due to problems with purity and crystalline structure. Accordingly, a thermally stable ceramic material with a high density and a high purity has not yet been obtained. For instance, although Japanese Laid Open Patent Application No. 64199/77 offers a method of adding compounds such as MgO in order to improve the insufficient sintering property of a synthetic hydroxyapaptite, this method is based on the introduction of elements other than Ca, P, O and H and so cannot provide a highly pure ceramic hydroxyapatite material.

In a different approach, M. Jarcho used a method by Haek (refer to Angew. Chem., 67:327 (1955) and Inorg. Synt. 7:63 (1963)) based on the following reaction:

$$5Ca(NO_3)_2 + 3(NH_4)_2HPO_4 + 4NH_3 + H_2O \rightarrow Ca_5(OH)\ (PO_4)_3 + 10NH_4NO_3$$

in which calcium nitrate is reacted with diammonium hydrogen phosphate while regulating the pH of the reaction system at 10 to 12 by the addition of ammonia to synthesize hydroxyapatite. The cake-like hydroxyapatite obtained by filtrating the reaction mixture can then be fired at a temperature of 1100 to 1200°C to provide a ceramic material.

It has been reported that a ceramic hydroxyapatite material having a mean crystal size of 0.2 to 3 microns and a density of 3.10 to 3.14 g/cm³ was obtained by this procedure (refer to U.S. Pat. No.

4,097,935, Japanese Patent Applications Nos. 40400/76, 94309/77 and "J. Material Sci.", 11:2027(1976)). However, it is disclosed in this literature that the ceramic hydroxyapatite material was partially decomposed to a by-product, whitlockite, when treated at a temperature above 1250°C for more than one hour. That is, the ceramic hydroxyapatite material obtained by the above-mentioned procedure is structurally unstable and accordingly has poor thermal stability. In addition, there is the difficulty of having to remove the ammonium nitrate by-product.

A different method for synthesizing hydroxyapatite utilizes the reaction of calcium hydroxide with phosphoric acid:—

$$5Ca(OH)_2 + 3H_3PO_4 \rightarrow Ca_5(PO_4)_3(OH) + 9H_2O.$$

This reaction could develop into an industrial method for producing hydroxyapatite because (1) no impurities are introduced and (2) only water is formed as a by-product, but Mooney has said ("Chem. Rev.", 61,433 (1961)) that it is difficult to obtain hydroxyapatite of the correct stoichiometric composition (theoretical Ca/P atomic ratio of 1.67:1) by the reaction, and that only hydroxyapatite having a Ca/P ratio of 1.50:1 corresponding to calcium triphosphate is obtainable. On the other hand, R. Wallaeys (refer to "Annales Chem. (Paris)" 7,808 (1952)) obtained a hydroxyapatite having a Ca/P atomic ratio of 1.61:1 to 1.67:1 by boiling the reaction mixture after the reaction was over in order to make the reaction proceed completely or making the reaction proceed to the neutral point to phenolphthalein while boiling.

However, the results of experiments on the method of Wallaeys carried out by the inventors of the present invention have shown that a dried filter cake of hydroxyapatite obtained by the Wallaeys method has a poor sintering property so that a ceramic material having a density of only about 3.11 $g/cm^3$ is obtained even by the hot-press method at a high temperature and, moreover, the ceramic material is coloured blue. The cause of the blue colouring is not yet known, but may be due to some structural defect and/or to the presence of a minute amount of impurities.

The inventors of the present invention have studied the relationship between the sintering property or the thermal stability of the ceramic material and the crystalline form and shape and the structure of synthetic hydroxyapatite, and have found that a ceramic material of excellent thermal stability, colour, purity and strength which has no structural defects and which does not decompose to form whitlockite even upon heating for one hour at a temperature of 1350°C is obtained by firing a dried filter cake of a synthetic hydroxyapatite prepared in a particular manner.

The invention provides a process for preparing hydroxyapatite, which comprises the steps of,

(a) converting calcium carbonate powder into calcium oxide by thermal decomposition in an inert atmosphere at a temperature of 800 to 1300°C for 0.5 to 10 hours:

(b) cooling the calcium oxide to a temperature lower than 500°C in the inert atmosphere to obtain extremely porous highly reactive calcium oxide:

(c) slaking the cooled calcium oxide with water while agitating thereby to obtain a fine calcium hydroxide milk of high purity; and

(d) reacting the calcium hydroxide with an aqueous solution of phosphoric acid while agitating in an inert atmosphere, thereby to obtain hydroxyapatite.

In the process of the present invention an aqueous suspension of minute particles of calcium hydroxide is formed in step (c) by bringing porous and minute particles of practically pure calcium oxide into reaction with an excess amount of water and when this calcium hydroxide is reacted in step (d) with an aqueous solution of phosphoric acid a milky reaction mixture containing the reaction product, a highly pure hydroxyapatite, is obtained.

The milky reaction mixture can then be filtered, the separated cake washed with water and dried to provide an aggregate of hydroxyapatite having the following properties:

Atomic ratio of Ca/P: 1.67:1 to 1.69:1,

Mean dimensions of hydroxyapatite crystals
length: 150 to 1200 Å
width: 50 to 400 Å, and
length/width ratio: 3:1 to 10:1
Pore volume: 0.2 to 0.8 $cm^3/g$
Mean pore-radius: 50 to 150 Å.

By firing the dried filter cake, the aggregate of hydroxyapatite, a ceramic material of hydroxyapatite is obtained which is colourless or semi-transparent and has the following physical properties:

Atomic ratio of Ca/P: 1.67:1 to 1.69:1,
Mean crystal size: 4 to 20 $\mu m$, and
Density: 3.14 to 3.16 $g/cm^3$.

The mean crystal size of this ceramic material is larger than that of known synthetic ceramic hydroxyapatite materials which indicates that the ceramic material of the present invention is prepared from purer raw materials than those used for preparing the known materials. In addition, the ceramic material of the invention has a thermal stability such that substantially no whitlockite is formed upon heating for at least one hour at 1350°C and this indicates that the ceramic material is substantially free of structural defects.

The invention will now be described in more detail:

Porous and minute particles of calcium oxide for use in the process of the present invention are obtained in step (a) of the process by thermally decomposing calcium carbonate powder. The calcium carbonate is preferably of high purity, say higher than 99.0%, preferably 99.8% or more, and in a microfine state. The conditions of the thermal decomposition of the calcium carbonate are: in an inert atmosphere, at a temperature of 800 to 1300°C, preferably 950 to 1200°C, for 0.5 to 10 hours, preferably one to five hours. Upon decomposition under the above-mentioned conditions, calcium carbonate is converted to a highly porous and reactive fine powder of calcium oxide while gaseous carbon dioxide is evolved.

If milder conditions of thermal decomposition were to be employed undecomposed calcium carbonate would remain in the calcium oxide produced. Not only is it difficult to remove any remaining calcium carbonate from the calcium oxide, but also any remaining calcium carbonate would cause structural defects and a reduction in the thermal stability of a ceramic hydroxyapatite material. If, however, the conditions of the thermal decomposition are more severe or if the product of the thermal decomposition were not cooled in step (b) in an inert atmosphere until the temperature of the product has fallen to 500°C, preferably to 200°C, the porosity and the reactivity of the calcium oxide produced would be impaired. The inert atmosphere for steps (a) and (b) should be an atmosphere which does not contain any components, such as gaseous carbon dioxide and ammonia, inducing a secondary reaction with the calcium oxide produced or with calcium hydroxide. It is effective to blow an inert gas such as nitrogen, helium or argon through the thermal decomposition zone in order to obtain the inert atmosphere because gaseous carbon dioxide generated is thus removed from the system.

An aqueous suspension of minute particles of calcium hydroxide is produced in step (c) by adding the calcium oxide from step (b) to a large amount of water with high speed stirring to provide turbulent flow under an inert atmosphere. The amount of water to be used should be at least enough to slake the calcium oxide but is not otherwise limited, however it usually is 10 to 100 parts by weight per part by weight of calcium oxide.

The slaking reaction is carried out with high speed stirring, preferably at a relatively low temperature for a relatively long time period and usually at 0 to 80°C, preferably 0 to 50°C, for 0.5 to 96 hours, preferably 1 to 24 hours.

The end point of the slaking reaction can be determined by X-ray diffraction analysis, wherein a part of the reaction product is withdrawn as a specimen and the disappearance of a $d_{200}$ line in the X-ray diffraction pattern of the specimen is taken as a sign that the reaction is complete.

The mean particle size of the calcium hydroxide resulting from step (c) of the process of the present invention is 0.05 to 0.1 $\mu$m and is far smaller than the mean particle size of one to ten $\mu$m of calcium hydroxide obtained by dispersing commercial calcium hydroxide in water.

The suspension of fine particles of calcium hydroxide is an important requisite for obtaining hydroxyapatite with a favorable sintering property described later.

Synthetic hydroxyapatite is formed in step (d) of the process of the present invention in a milky state by adding an aqueous solution of phosphoric acid in an amount corresponding to a Ca/P atomic ratio of 1.67:1 to 1.69:1 to the aqueous suspension of minute particles of calcium hydroxide under an inert atmosphere. In the preparation of the hydroxyapatite, it is preferable that high speed stirring is continued after completing step (c) and that the aqueous solution of phosphoric acid is added to the system with stirring.

The phosphoric acid is usually an aqueous one to ten percent by weight solution and the solution is slowly added to the calcium hydroxide suspension. In this connection, the high speed stirring referred to herein means stirring which causes turbulent flow of the liquid in the reaction system.

The reaction temperature of step (d) suitably is 0 to 50°C. At a lower temperature, the viscosity of the suspension would be so high that a uniform reaction would become difficult, while at a higher temperature the sintering property of the aggregate of hydroxyapatite is impaired. Thus, the reaction temperature of step (d) is an extremely important factor affecting the size of crystals, concerning the sintering property, or affecting the easily sedimenting property which will be described later. The reaction is usually carried out for 0.5 to 200 hours, preferably for 5 to 100 hours. The reaction mixture is highly alkaline having a pH of about 13 initially, falling to 8 to 9 at the completion of the reaction.

Although the synthetic hydroxyapatite in the reaction system assumes a milky state, it has excellent sedimentability and is easiliy separable by filtration. The synthetic hydroxyapatite crystals are, as has been described, 50 to 400 Å in width and 150 to 1200 Å in length, and have a length to width ratio of 3 to 10. These dimensions are clearly unlike those of the plate-like crystals about 200 Å in width and about 200 Å in length obtained by the aforementioned reaction between calcium nitrate and diammonium hydrogen phosphate. The easy sedimentability of the synthetic hydroxyapatite obtained

by the process of the present invention is considered to be due to such a size of crystals, their shape and form and further to the state of electric charge on the surface of the crystals. Moreover, these factors presumably contribute to the formation of the aggregate, or filter cake, of hydroxyapatite of the present invention, which has a favorable sintering property.

An aggregate of hydroxyapatite according to the present invention is obtained by filtering the synthetic hydroxyapatite obtained by the above-mentioned process, suitably to a water content of 0 to 2% by weight. The thus obtained filter cake or aggregate of hydroxyapatite has a pore volume of 0.2 to 0.8 $cm^3/g$ and pore radius 50 to 150 Å, and comprises hydroxyapatite crystals having a Ca/P atomic ratio of 1.67:1 to 1.69:1, crystal size of 50 to 400 Å in width, 150 to 1200 Å in length, and width to length ratio of 3:1 to 10:1, and these properties affect the specific properties of the ceramic hydroxyapatite material.

The aggregate of hydroxyapatite according to the present invention has a favorable sintering property and when fired as it is, a ceramic material having excellent properties is obtained.

Naturally, conventional methods for molding and firing the aggregate are applicable in preparing the ceramic material according to the present invention, for instance the aggregate may be molded using a metal or rubber mold and then baked, or the aggregate may be molded and fired by a hot press method, or a molded cake can be fired under a reduced pressure.

A ceramic material which is highly translucent, highly pure, highly dense and highly stable thermally is obtainable by firing under reduced pressure after pressure-molding an aggregate of hydroxyapatite according to the present invention. The conditions of firing suitably are a temperature of 850 to 1400°C, preferably 1250 to 1400°C, for 0.5 to 5 hours, preferably 1 to 3 hours. It has been noticed that the growth of the hydroxyapatite crystals during the sintering by firing in accordance with the invention is remarkably greater than when conventional hydroxyapatite is fired. This is presumably due to the above-mentioned specific properties of the aggregate of hydroxyapatite and its favorable sintering property.

The ceramic hydroxyapatite material prepared according to the present invention has the following properties of:

a) atomic ratio Ca/P: 1.67:1 to 1.69:1;
b) mean size of crystals: 4 to 20 $\mu$m;
c) density: 3.14 to 3.16 $g/cm^3$ and
d) thermal stability: no formation of whitlockite detectable after heating for one hour at 1350°C.

While known ceramic hydroxyapatite materials form whitlockite heated at a temperature higher than 1200°C or 1300°C, the ceramic material according to the present invention is extremely stable at temperatures higher than 1300°C, and the size of the crystals of the material according to the present invention is larger than that of the known ceramic hydroxyapatite materials, so that the former is clearly different from the latter.

The ceramic material according to the present invention has excellent stability within a living body presumably due to the absence of structural defects.

The thermal stability of the above-mentioned ceramic material is confirmed by the determination of X-ray diffraction pattern after sintering the material of one hour at a temperature of 1350°C.

The process of the present invention is extremely effective as an industrial method and can be used to provide a ceramic hydroxyapatite material which is chemically and physico-structurally pure and stable enough to be used within living bodies and useful as an implant material such as artificial dental roots and artificial bones.

The practical application of the ceramic hydroxyapatite material as an implant material, particularly as an artificial dental root material, will next be described. The ceramic material may be solely utilised for an implant material.

An implant material according to the present invention is prepared, with consideration to processability, ease of handling and mechanical strength, by molding a mixture of the ceramic hydroxyapatite material having a particle size smaller than 1000 $\mu$m, preferably 0.01 to 100 $\mu$m, and an organic matrix, or by impregnating a porous ceramic material of the present invention with an organic bonding material. In preparing the implanting material, it is important that the ratio of the area of the hydroxyapatite phase at the working surface (that is the surface which in use contacts bone) of the implant material to that of the organic bonding material is 5:95 to 70:30, preferably 10:90 to 60:40. When the hydroxyapatite phase provides more than 70% of the surface area of the working surface, the adhesion of the implant to natural bone is too great so that the natural bone tends to be damaged when the implant is removed. However, when the hydroxyapatite phase provides less than 5% of the surface area of the worming surface, the implant tends to be naturally rejected. The above-mentioned ratio has been decided in consideration of the durability and adhesiveness of an artificial dental root.

The organic bonding material should have a long active life if it is to be used in a long-term implant in a living body and should not degrade the cells of the living body. Bonding materials which may be used include polcondensates of bisphenol A and glycidyl methacrylate, methyl methacrylate

polymers, 2-hydroxyethyl methacrylate polymers, triethyleneglycol dimethacrylate polymers, ethylene polymers, sulfone polymers, polyamides, polyesters, tetrafluoroethylene polymers, vinylidene fluoride polymers and polycarbonates. These materials can be used singly or as mixtures of two or more thereof. The polymers can be homopolymers, or copolymers of the appropriate monomer with one or more copolymerisable monomer.

In addition, the ceramic material of the present invention can be used as a component of a material to be used within a living body such as a co-existent-type sintered material with an organic substance such as cellulose or collagen, a porous material prepared by boring, a composition prepared by impregnating with an organic resin, or impregnating and polymerizing an organic monomer into the porous material, a composite of the powdery ceramic material with an organic or inorganic matrix, or a combination of these.

The hydroxyapatite according to the present invention can also be used as a filling material for a chromatographic column.

The present invention will be described in more detail by reference to the following Examples:

## Example 1

600 g of powdery calcium carbonate of reagent grade (G.R.) were introduced into an electric furnace under a flow of gaseous nitrogen supplied at a flow rate of one litre/min. and heated to a temperature of 1000°C at a rate of 3°C/min. After heating for 3 hours at 1000°C to decompose the calcium carbonate, the furnace was cooled to 200°C under the flow of gaseous nitrogen at a rate of one litre/min to provide 335 g of calcium oxide in a yield of 99.7%. It was confirmed by X-ray diffraction analysis that the calcium oxide did not contain calcium carbonate. The purity of the product determined by the EDTA-method was 99.8%. According to microscopical observation, the crystal shape of the obtained calcium oxide was similar to that of the raw material, calcium carbonate, and the crystal of the calcium oxide was porous.

Into a 17-litre three necked porcelain enamelled tank provided with a heater, a temperature-controller and a thermometer, 6 litres of de-aired distilled water were introduced, the atmosphere in the tank was substituted by nitrogen while agitating with a stirrer operating at 350 rpm. Then, 280 g (5 mol) of the calcium oxide were slowly added within 10 minutes, and then made to react for 5 hours at 50°C, and for 15 hours at room temperature under the atmosphere of gaseous nitrogen to obtain a suspension of calcium hydroxide of 0.075 $\mu$m in mean particle diameter.

Next, while keeping the suspension in turbulent flow by agitation at 3000 rpm, three moles of an aqueous 3.5% solution of phosphoric acid prepared from 85% phosphoric acid, were added over 30 minutes, and then the reaction was continued for 48 hours at a temperature of 20°C. After the reaction was over, the resulting suspension was filtered under pressure and the residue was washed with water and dried at 150°C for 16 hours.

Thus, 497 g of a dried filter cake of hydroxyapatite were obtained and named as Specimen No. 1. The properties of the dried material, that is the aggregate of a hydroxyapatite of the present invention are shown in Table 1.

## Example 2

Another aggregate of hydroxyapatite of the present invention named as Specimen No. 2 was prepared by the same procedure as in Example 1 except that the reaction with phosphoric acid was carried out at 40°C for 24 hours as compared with 20°C and 48 hours in Example 1. The properties of Specimen No. 2 are also shown in Table 1.

## Comparative Example 1

An aqueous dispersion prepared by adding 5 mols of powdery calcium hydroxide of reagent grade (G.R.), into 6 litres of de-aired distilled water was introduced into the porcelain enamelled tank used in Example 1, and while agitating at 3000 rpm under the substituted nitrogen atmosphere by a flow of gaseous nitrogen, 3 moles of an aqueous 3.5% solution of phosphoric acid were slowly added to the dispersion. After carrying out a reaction at a temperature of 70°C for 24 hours, the same procedures as in Example 1 were taken to obtain an aggregate of hydroxyapatite named as Specimen No. 3, the properties of which are also shown in Table 1.

## Comparative Example 2

Another aggregate of hydroxyapatite named Specimen No. 4 was prepared by the following known method and its properties were also shown in Table I:

Diammonium hydrogen phosphate (160 g) was dissolved in distilled water (3 litres) and an aqueous 28% ammonia solution (1700 ml) was added to the solution to adjust the pH to 11 to 12. Distilled water was further added to it to dissolve the thus precipitated diammonium hydrogen phosphate. This solution was poured over 30 minutes into a solution prepared by dissolving 477 g of calcium nitrate in 188 ml of distilled water adjusted to pH of 12 by an addition of 60 ml of a concentrated aqueous ammonia solution and kept at a temperature of 20°C with high speed stirring. The mixture was further diluted with distilled water until the total volume was 7.0 litres. After boiling the thus diluted solution for 10 min, it was left at room temperature for 20 hours.

The thus produced gelatinous material was filtered using a Buchner funnel under slightly reduced pressure, washed with water while kept on the filter, and when cracks appeared on the surface of the filter cake, a higher vacuum was applied for 2 hours. The filter cake was dried at 150°C for 15 hours to provide 197 g of an aggregate of hydroxyapatite.

Example 3

Respective specimens of the aggregates of hydroxyapatite obtained by Examples 1 and 2 and Comparative Examples 1 and 2 were placed in an electric furnace and heated to a temperature of 1350°C at a rate of 3°C/min. After keeping at that temperature for one hour, each specimen was removed from the furnace to be cooled to room temperature. The thus obtained ceramic materials had the respective properties shown in Table 2.

Samples of each ceramic material were crushed to pieces of about 5 mm in size, and after immersing the pieces in a 0.1% aqueous solution of neutral red at room temperature for 15 hours, the pieces were washed with water and dried to examine the coloration of their crushed surfaces. The results of the examination showed that although no coloration was observed on Specimens No. 1 and No. 2 of Examples 1 and 2, a coloration was observed on Specimen No. 4 of Comparative Example 2. Since Specimen No. 3 showed a blue colour before immersing into the solution of the dyestuff, it could not be compared. From these results it was clear that the ceramic material of the present invention differs from the known ceramic material.

Example 4

Twenty grams of the aggregate of hydroxyapatite of the present invention obtained in Example 1, Specimen No. 1, were fired at a temperature of 1250°C under a reduced pressure of $10^{-2}$ mmHg (1.33 Pa) for one hour, and cooled to 200°C under the reduced pressure to prepare a ceramic material. The thus prepared ceramic material was white in colour, transparent and showed a density of 3.16 g/cm$^3$.

Example 5

A sintering test was carried out on the aggregates of hydroxyapatite of Specimen No. 1 of Example 1 and Specimen No. 4 of Comparative Example 2. 20 g samples of each Specimen were baked at each temperature of 1200, 1250, 1300 and 1350°C for one hour, and the amount of whitlockite formed within the fired material was determined by X-ray diffraction analysis. The results are shown in Table 3. As is seen in Table 3, the difference between the ceramic material according to the present invention and the known ceramic material is clear.

Example 6

The ceramic material prepared in Example 3 from Specimen No. 1 was pulverized and the fraction passed through a sieve of 200 mesh (aperture 74 $\mu$m) was collected. The thus collected powder was mixed with a 6:4 by weight mixture of copolymer of bisphenol A and glycidyl methacrylate and monomeric methyl methacrylate at a volume ratio (calculated by the respective weights and densities of both components) of 1:1, and after the further addition of 0.05% by weight of benzoyl peroxide as a polymerization initiator, the mixture was mixed to be uniform, and after pouring into a glass tube of 5 mm inner diameter and debubbling, a polymerization was carried out at 80°C for 2 hours on the mixture to obtain a composition of hydroxyapatite. After processing the composition into a cylinder of 3.5 mm in diameter and 10 mm in length it was implanted into the drilled hole of the jaw-bone of an adult dog just after a tooth extraction. The implant was not naturally removed even after 3 months of implantation. After 6 months of implantation, the jaw bone of the dog was cut off to be examined by an optical microscope and roentgenography. It was found that the implanted part had healed normally and new bone-tissue had formed in the gap between the implant and the jaw-bone.

TABLE 1:
Properties of Aggregates of hydroxyapatite

| Classification | Specimen No. | Reaction time (hour) temp. (°C) | Yield (%) | Elementary analysis Ca(%) | Elementary analysis P(%) | Elementary analysis Ca/P | Size of crystal length L(Å) | Size of crystal width W(Å) | Size of crystal L/W | Pore volume[1] (ml/g) | Pore-radius[2] (A) |
|---|---|---|---|---|---|---|---|---|---|---|---|
| Theoretical | | | | 39.89 | 18.43 | 1.67 | | | | | |
| Present invention | 1 | 48<br>20 | 99 | 39.75 | 18.43 | 1.67 | 300 | 80 | 3.75 | 0.47 | 100 |
| | 2 | 24<br>40 | 99 | 39.80 | 18.46 | 1.67 | 900 | 200 | 4.50 | 0.65 | 140 |
| Comparative | 3 | 24<br>70 | 99 | 39.78 | 18.45 | 1.67 | 2300 | 350 | 6.57 | 0.88 | 400 |
| | 4 | 24<br>20 | 98 | 39.50 | 18.40 | 1.66 | 200 | 200 | 1.00 | 0.45 | 90 |

Note: 1) and 2) determined by POROSIMETRO Model 70 (manufactured by Acom Co.)

TABLE 2:
Properties of Hydroxyapatite Ceramic Materials

| Classification | Raw material Specimen No. | Elementary analysis | | | Mean size of crystal ($\mu m$) | Density ($g/cm^3$) | Colour | Thermal stability[1)] |
|---|---|---|---|---|---|---|---|---|
| | | Ca(%) | P(%) | Ca/P | | | | |
| Theoretical | | 39.89 | 18.43 | 1.67 | | | | |
| Present invention | 1 | 39.87 | 18.51 | 1.67 | 11 | 3.16 | white translucent | no decomposition |
| | 2 | 39.86 | 18.48 | 1.67 | 5 | 3.15 | white translucent | no decomposition |
| Comparative example | 3 | 39.85 | 18.47 | 1.67 | 2.5 | 3.02 | light blue | no decomposition |
| | 4 | 38.34 | 18.40 | 1.61 | 3 | 3.12 | white | decomposition observed |

Note: 1) evaluated by the presence of absence of the formation of whitlockite in the specimen heated for one hour at a temperature of 1,350°C.

TABLE 3:
Results of Sintering Test: amount of whitlockite

| Classification Raw Material | | Temperature of firing (°C) | | | |
|---|---|---|---|---|---|
| | | 1200 | 1250 | 1300 | 1350 |
| Present invention | Specimen No. 1 | 0 | 0 | 0 | 0 |
| Comparative example | Specimen No. 4 | 6.0 | 41.5 | 41 | 42 |

## Claims

1. A process for preparing hydroxyapatite by the reaction of calcium hydroxide with phosphoric acid, characterised by

(a) converting calcium carbonate powder into calcium oxide by thermal decomposition in an inert atmosphere at a temperature of 800 to 1300°C for 0.5 to 10 hours;

(b) cooling the calcium oxide to a temperature lower than 500°C in the inert atmosphere;

(c) slaking the cooled calcium oxide with water while agitating thereby to obtain calcium hydroxide; and

(d) reacting the calcium hydroxide with an aqueous solution of phosphoric acid while agitating in an inert atmosphere thereby to obtain hydroxyapatite.

2. A process according to claim 1, characterised in that an aqueous suspension of calcium hydroxide is reacted with the aqueous solution of phosphoric acid in step (d).

3. A process according to claim 1 or 2, characterised in that the calcium hydroxide is reacted with the aqueous solution of phosphoric acid in step (d) at a temperature of 0 to 50°C.

4. A process according to claim 1, 2 or 3 characterised in that the reaction product from step (d) is filtered and dried to a water content of 2% by weight or less.

5. A filtered, dried cake of synthetic hydroxyapatite characterised by having a three-dimensional porous structure with an average pore radius of 50 to 150 Å (500 to 1500 nm) and a pore volume of 0.2 to 0.8 $cm^3/g$, said cake comprising crystalline hydroxyapatite having an atomic ratio of calcium to phosphorus of 1.67:1 to 1.69:1 in which the crystals have a length of 150 to 1200 Å (1500 to 12000 nm), a width of 50 to 400 Å (500 to 4000 nm), and a length to width ratio of 3:1 to 10:1 produced by the process of claim 1, 2, 3 or 4.

6. A ceramic material comprising synthetic hydroxapatite and having an atomic ratio of calcium to phosphorus of 1.67:1 to 1.69:1 characterised by average crystal size of 4 to 20 $\mu m$, a density of 3.14 to 3.16 $g/cm^3$ and a thermal stability such that substantially no whitlockite is formed upon heating for at least one hour at a temperature of 1350°C, said ceramic material being colourless or semi-transparent and being obtained by sintering a dried, filtered cake of synthetic hydroxyapatite as claimed in claim 5.

7. An implant material comprising a ceramic hydroxyapatite material and biologically acceptable bonding material characterised in that the ceramic material is as claimed in claim 6.

8. An implant material according to claim 7, characterised in that the ceramic material forms from 5 to 70% of the surface area of the implant material.

9. An implant material according to claim 7 or 8, characterised in that the bonding material comprises at least one of polycondensates of bisphenol A and glycidyl methacrylate, methyl methacrylate polymers, 2-hydroxyethyl methacrylate polymers, triethyleneglycol dimethacrylate polymers, ethylene polymers, sulfone polymers, polyamides, polyesters, tetrafluoroethylene polymers, vinylidene fluoride polymers and polycarbonates.

## Revendications

1. Procédé de préparation d'hydroxyapatite par la réaction d'hydroxyde de calcium avec de l'acide phosphorique, caractérisé en ce que l'on

a) transforme de la poudre de carbonate de calcium en oxyde de calcium par décomposition thermique dans une atmosphère inerte à une température de 800 a 1300°C pendant 0,5 à 10 heures;

b) refroidit l'oxyde de calcium à une température inférieure à 500°C dans l'atmosphère inerte;

c) éteint l'oxyde de calcium refroidi avec de l'eau tout en agitant pour obtenir ainsi de l'hydroxyde de calcium; et,

d) fait réagir l'hydroxyde de calcium avec une solution aqueuse d'acide phosphorique tout en agitant dans une atmosphère inerte pour obtenir ainsi l'hydroxyapatite.

2. Procédé suivant la revendication 1, caractérisé en ce que l'on fait réagir une suspension aqueuse d'hydroxyde de calcium avec la solution aqueuse d'acide phosphorique dans l'étape d).

3. Procédé suivant les revendications 1 ou 2, caractérisé en ce que l'on fait réagir l'hydroxyde de calcium avec la solution aqueuse d'acide phosphorique dans l'étape d) à une température de 0 à 50°C.

4. Procédé suivant la revendication 1, 2 ou 3, caractérisé en ce que le produit de la réaction de l'étape d) est filtré et séché jusqu'à une teneur en eau de 2% en poids ou moins.

5. Gâteau filtré sec d'hydroxyapatite synthétique caractérisé en ce qu'il à une structure poreuse tridimensionnelle présentant un rayon de pores moyen de 50 à 150 Å (500 à 1.500 nm) et un volume de pores de 0,2 à 0,8 $cm^3/g$, ledit gâteau contenant de l'hydroxyapatite cristalline ayant un rapport atomique du calcium au phosphore de 1,67:1 à 1,69:1 dans lequel les cristaux ont une longueur de 150 à 1.200 Å (1.500 à 12.000 nm), une largeur de 50 à 400 Å (500 à 4.000 nm) et un rapport de longueur à la largeur de 3:1 à 10:1, produit par le procédé des revendications 1, 2, 3 ou 4.

6. Matériau céramique contenant de l'hydroxyapatite synthétique et ayant un rapport atomique du calcium au phosphore de 1,67:1 à 1,69:1, caractérisé par une taille moyenne des cristaux de 4 à 20

$\mu$m, une densité de 3,14 à 3,16 g/cm$^3$ et une stabilité thermique telle qu'il ne se forme sensiblement pas de whitlockite par chauffage pendant au moins 1 heure à une température de 1350°C, ledit matériau céramique étant incolore ou semi-transparent et étant obtenu par frittage d'un gâteau filtré sec d'hydroxyapatite synthétique tel que revendiqué dans la revendication 5.

7. Matériau d'implant contenant un matériau céramique d'hydroxyapatite et un matériau liant biologiquement acceptable, caractérisé en ce que le matériau céramique est tel que revendiqué dans la revendication 6.

8. Matériau d'implant suivant la revendication 7, caractérisé en ce que le matériau céramique forme de 5 à 70% de la surface du matériau d'implant.

9. Matériau d'implant suivant la revendication 7 ou 8, caractérisé en ce que le matériau liant comprend au moins l'un parmi les polycondensats de bisphénol A et glycidyl-méthacrylate, polymères de méthylméthacrylate, polymères de 2-hydroxyéthyl-méthacrylate, polymères de triéthylèneglycol diméthacrylate, polymères d'éthylène, polymères de sulfone, polyamides, polyesters, polymères de tétrafluoroéthylène, polymères de fluorure de vinylidène et polycarbonates.

**Patentansprüche**

1. Verfahren zur Herstellung von Hydroxyapatit durch Umsetzung von Calciumhydroxid mit Phosphorsäure, gekennzeichnet durch

(a) Überführung von Calciumcarbonat-Pulver in Calciumoxid durch thermische Zersetzung in einer inerten Atmosphäre bei einer Temperatur von 800 bis 1300°C für 0,5 bis 10 Stunden;

(b) Abkühlen des Calciumoxids auf eine Temperatur unterhalb von 500°C in der inerten Atmosphäre;

(c) Löschen des gekühlten Calciumoxids mit Wasser unter Rühren, um Calciumhydroxid zu erhalten, und

(d) Umsetzen des Calciumhydroxids mit einer wäßrigen Lösung von Phosphorsäure unter Rühren in einer inerten Atmosphäre, um Hydroxyapatit zu erhalten.

2. Verfahren nach Anspruch 1, dadurch gekennzeichnet, daß eine wäßrige Suspension von Calciumhydroxid mit der wäßrigen Phosphorsäure-Lösung in Schritt (d) umgesetzt wird.

3. Verfahren nach Anspruch 1 oder 2, dadurch gekennzeichnet, daß das Calciumhydroxid mit der wäßrigen Phosphorsäure-Lösung in Schritt (d) bei einer Temperatur von 0 bis 50°C umgesetzt wird.

4. Verfahren nach Anspruch 1, 2 oder 3, dadurch gekennzeichnet, daß das Reaktionsprodukt aus Schritt (d) filtriert und zu einem Wassergehalt von 2 Gew.-% oder weniger getrocknet wird.

5. Filtrierter, getrockneter Kuchen aus synthetischem Hydroxyapatit, dadurch gekennzeichnet, daß er eine dreidimensionale Porenstruktur mit einem durchschnittlichen Porenradius von 50 bis 150 Å (500 bis 1500 nm) und einem Porenvolumen von 0,2 bis 0,8 cm$^3$/g besitzt, wobei der Kuchen kristallinen Hydroxyapatit mit einem Atomverhältnis von Calcium zu Phosphor von 1,67:1 bis 1,69:1 besitzt, worin die Kristalle eine Länge von 150 bis 1200 Å (1500 bis 12000 nm), eine Breite von 50 bis 400 Å (1500 bis 4000 nm) und ein Verhältnis von Länge zu Breite von 3:1 bis 10:1 aufweisen, hergestellt durch das Verfahren nach Anspruch 1, 2, 3 oder 4.

6. Keramisches Material, welches synthetischen Hydroxyapatit enthält und ein Atomverhältnis von Calcium zu Phosphor von 1,67:1 bis 1,69:1 aufweist, gekennzeichnet durch eine mittlere Kristallgröße von 4 bis 20 $\mu$m, eine Dichte von 3,14 bis 3,16 g/cm$^3$ und eine derartige thermische Stabilität, daß im wesentlichen kein Whitlockit bei Erhitzen für mindestens eine Stunde auf eine Temperatur von 1350°C gebildet wird, wobei das keramische Material farblos oder halb-transparent ist und durch Sintern eines getrockneten, filtrierten Kuchens von synthetischem Hydroxyapatit gemäß Anspruch 5 erhalten wird.

7. Implantat-Material, enthaltend ein keramisches Hydroxyapatit-Material und biologisch verträgliches Bindemittel, dadurch gekennzeichnet, daß das Keramikmaterial der Definition in Anspruch 6 entspricht.

8. Implantat-Material nach Anspruch 7, dadurch gekennzeichnet, daß das Keramik-Material 5 bis 70% des Oberflächenbereiches des Implantat-Materials bildet.

9. Implantat-Material nach Anspruch 7 oder 8, dadurch gekennzeichnet, daß das Bindemittel mindestens eines der Polykondensate von Bisphenol A und Glycidylmethacrylat, Methylmethacrylatpolymeren, 2-Hydroxyethylmethacrylatpolymeren, Triethylenglykoldimethacrylatpolymeren, Ethylenpolymeren, Sulfonpolymeren, Polyamiden, Polyestern, Tetrafluoroethylenpolymeren, Vinylidenfluoridpolymeren und Polycarbonaten enthält.